Europäisches Patentamt

European Patent Office

Office européen des brevets

(11) Publication number: **0 152 062**
**A2**

(12) **EUROPEAN PATENT APPLICATION**

(21) Application number: **85101274.0**

(22) Date of filing: **07.02.85**

(51) Int. Cl.⁴: **A 61 M 5/14**

(30) Priority: **13.02.84 US 579827**

(43) Date of publication of application:
**21.08.85 Bulletin 85/34**

(84) Designated Contracting States:
**DE GB**

(71) Applicant: **ABBOTT LABORATORIES**
**14th Street and Sheridan Road North St**
**North Chicago Illinois 60064(US)**

(72) Inventor: **Gross, James Robert**
**1080 Independence Drive**
**Bartlett Illinois 60103(US)**

(74) Representative: **Modiano, Guido et al,**
**MODIANO, JOSIF, PISANTY & STAUB Modiano &**
**Associati Via Meravigli, 16**
**I-20123 Milan(IT)**

(54) **Improved cannula hub device.**

(57) An improved cannula and hub assembly has a recess in the hub which provides for the mounting of a placement member. Projecting wing-like segments which protrude from the placement member provide surfaces on which force may be exerted when inserting the cannula into a patient. Once the cannula has been properly placed within the patient the placement member may be removed from the cannula hub.

FIG. 1

## Background of the Invention

This invention relates to an improved injection device or cannula hub device. More specifically this invention relates to an improved cannula hub gripping and positioning system.

Cannulas specifically designed for inserting medication or anesthesia into a patient, particularly in the area of the patient's spinal column, are well known in the art. Typical of these devices are the Touhy cannula which is characterized by a pointed offset tip on the end of the cannula which is to be inserted through the spinous ligaments into the epidural space of a patient. Such cannulas are normally mounted in a cannula hub. The cannula hub provides for attachment to and fluid communication with a syringe or fluid administration system or for the passage of a thin catheter into the epidural space of a patient.

Proper placement of a cannula such as a Touhy type needle within the epidural space of a patient is a delicate operation usually requiring both extensive training and great expertise on the part of health care personnel. In particular, most practitioners who insert cannulas into a patient's spinal column first cause the cannula to move through the tough spinous ligaments by exerting force on the cannula hub. The positioning of the tip of the cannula within the epidural space is achieved by stopping the forward movement of the cannula when a relaxation of resistance to further advancement of the tip of the cannula is felt at the cannula hub. This relaxation of resistance to movement occurs when the tip of the cannula penetrates the ligmentum flavum which surrounds the epidural space of the patient. This tactile method of epidural cannula insertion and placement presents several problems with regard to control of the position and travel of the cannula.

First, any slight movement from a direct path to the epidural space may cause the tip of the cannula to remain in the spinous ligaments and miss the epidural space of the patient. This problem is accentuated in older or muscular patients where the spinous ligaments may be exceptionally tough or fibrous, thus requiring a great deal of force to be applied to the cannula hub. Second, mere finger contact with the sides of the cannula hub will not provide sufficient frictional force to insert the cannula properly into the epidural space of a patient having tough or fibrous spinous ligaments. Such finger contact with the cannula hub is critical to proper cannula placement because the loss of resistance to movement occurring when the tip of the cannula passes through the ligmentum flavum into the epidural space must be accurately sensed at the cannula hub to assure proper placement of the cannula tip just past the ligmentum flavum in the epidural space.

Placement wings to facilitate manipulation and placement of cannulas are typically used with intravenous catheter systems. These wings provide an extra surface for health care personnel to use when manipulating a cannula for effecting venipuncture. Typical of these placement wings are those illustrated in U.S. Patent 3,064,648 to Bujan; U.S. Patent 3,538,915 to Frampton; U.S. Patent 3,598,118 to Warren; U.S. Patent 3,782,383 to Thompson et al.; and U.S. Patent 4,392,856 to Lichtenstein. These five patents illustrate methods by which the cannula itself may be gripped, moved and positioned by the flexing of wings about the cannula. In situations wherein these wing-type attachments are used on a cannula, the problem of moving the cannula through fibrous material is normally not encountered. Consequently, a need exists within the art to provide a cannula and hub assembly wherein improved cannula and hub gripping means are provided which are sufficient to facilitate insertion of the cannula through tough and fibrous portions of the body, yet still allow for detection of loss of resistance to the movement of the cannula. One example of an attempt to solve this problem is given in U.S. Patent No. 2,794,435 to Stevens wherein a flange is formed as part of a cannula hub. The Stevens '435 patent however, does not recognize that once the cannula has been inserted, the permanently mounted surface which is used for the application of force will provide a protrusion that can be bumped or snared by other equipment. Consequently, a need exists in the art to provide a system whereby the user of a cannula and hub assembly is assisted in inserting the cannula into the body of a patient but is provided a cannula and hub assembly that is free of obstructions which may be accidentally contacted or ensnared by other equipment.

It is therefore an advantage of this invention to provide an improved cannula and hub device which allows the cannula to be easily inserted through tough or fibrous portions of tissue within the body of a patient. Another advantage of the device of this invention is to provide a removable placement member that can be removed from the cannula hub so that it will not provide any obstruction or hindrance to health care personnel while the cannula is being used to apply medication or anesthesia to a patient.

Summary of the Invention

The cannula and hub assembly includes a removable placement member having three sections; specifically: first and second projecting segments and a connecting section. The connecting section and the projecting segments are arranged to form an upside-down U-shaped opening such as in a saddle. The upside-down U-shaped opening fits in a frictional or snap-fit manner within a recess in the cannula hub which mounts the cannula. The recess is formed transversely within the cannula hub so that the removable placement member may be positioned for most effective control of the cannula hub. Once mounted on the cannula hub the movement of the cannula may be guided by first and second projecting segments while they are being used as surfaces for pushing the cannula through fibrous ligaments such as those normally found in the spinal area of a patient. It is the positioning of the planar surfaces on the first and second projecting segments substantially transverse to the longitudinal axis of the cannula that allows movement force to be applied in a plane other than one substantially parallel to the cannula, thus giving a mechanical advantage and guide means to the user of the cannula. When the cannula has been properly positioned such as within the epidural space of a patient, the placement member may be removed from the cannula hub. Being removable, the cannula hub is free of any protrusions which may be bumped or entangled with other equipment or clothing.

## Brief Description of the Drawings

Other features and advantages of the present invention will become more fully apparent from the following detailed description of the embodiments, the appended claims and the accompanying drawings in which:

FIGURE 1 is an assembly view in perspective of the preferred embodiment of the cannula and hub assembly of the present invention.

FIGURE 2 is a view in horizontal section taken along line 2-2 of FIGURE 1.

FIGURE 3 is a view similar to FIGURE 1 of an alternative embodiment of the device of the present invention.

## Detailed Description of the Preferred Embodiment

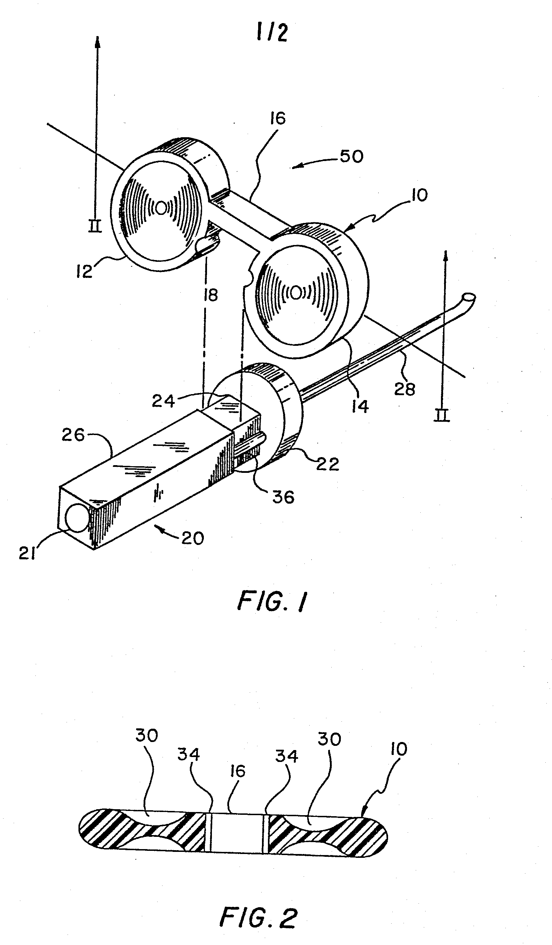

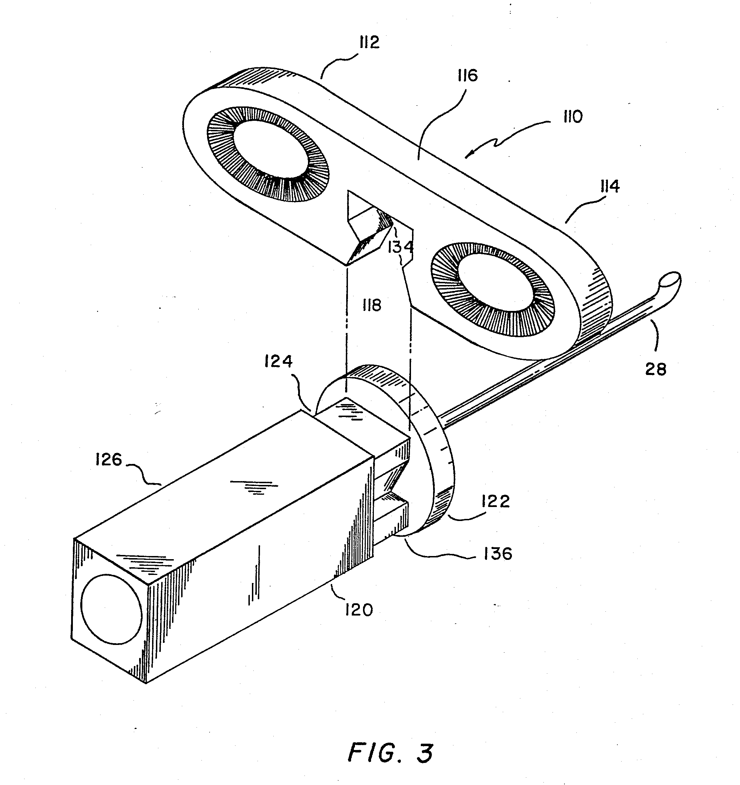

The preferred embodiment of the present invention is illustrated in FIGURES 1 and 2. An improved cannula and hub assembly generally 50 includes a removable placement member generally 10 having two projecting segments or wing-like members 12 and 14. The two projecting segments 12 and 14 are connected to each other by way of a connecting section or beam 16 and are positioned in a substantially transverse manner with respect thereto. The inner surfaces of projecting segments 12 and 14 and connecting section 16 form an upside-down U-shaped opening 18 in removable placement member 10 which is designed to fit into recess 24 provided in part by flange 22 of cannula hub assembly generally 20 in a frictional or snap-fit manner. Passage 21, which may incorporate a luer taper for interfitment with other devices, extends through the length of cannula hub 26 and is in fluid communication with the bore 27 of cannula 28. As best seen in FIGURE 2 depressions 30 may be formed within the planar surfaces of projecting segments 12 and 14. The use of these depressions 30 will be more fully explained in the description of operation which follows. Within opening 18 are found two notches 34. These notches 34 are complementary to ridges 36 formed on the sides of recess 24 in hub body 20. The notch 34 and ridge 36 latching system holds removable placement member 10 securely in place on cannula hub body 20 in a frictionally tight manner. This latching system may assume many different configurations as more particularly shown in FIGURE 3.

In FIGURE 3, an alternate means of attaching removable placement member generally 110 to cannula hub body 120 is shown. Parts having the same or similar construction, function and relative location as in the preferred embodiment are given numbers in the "100" series. More particularly, angled ledges 134 on the sides of opening 118 are formed as a part of connecting section 116 between projecting segments 112 and 114. Complementary bevels 150 to ledges 134 are formed within recess 124 on either side of cannula hub body 120 to receive connecting section 116 in a frictionally tight manner. This means of securing the position of removable placement member 110 to cannula hub body 120 is exemplary of but one of many different configurations that may be used to secure removable placement member 10 or 110 to cannula hub body 20 or 120.

Description of Operation

The removable placement member 10 is attached to cannula hub body 20 before or after cannula 28 is inserted into a patient. Once connected to cannula hub body 20 removable placement member 10 provides a surface generally transverse to the longitudinal axis of cannula 28 on which finger pressure may be exerted in order to advance cannula 28 through fibrous tissue. Recesses 30 are provided in projecting segments 12 and 14 to assist in the placement of the users fingers with respect to the cannula hub 20 and removable placement member 10. As best seen in FIGURE 2, removable placement member 10 is reversible with respect to hub body 20 as it is the same on both sides. When finger pressure is exerted on projecting segments 12 and 14, force is transmitted by removable placement member 10 to the inner surface of flange 22. This transmitted force then causes the tip 27 of cannula 28 to advance easily, even through fibrous tissue to the point where medicament is to be placed in the body of a patient. When the desired point has been reached by the tip of cannula 28, the removable placement member 10 may be removed so that it will not interfere with connections made to the cannula hub 20 at opening 21 or equipment used nearby. The alternative embodiment shown in FIGURE 3 operates in substantially the same way as the preferred embodiment shown in FIGURE 1.

The device of the present invention may be constructed from suitable medical grade plastics such as ABS or polystyrene. More particularly, removable placement members 10 and 110 should be molded of medical grade plastic which is sufficiently flexible to allow them to be frictionally attached to and then removed from cannula hub 20. Cannula hub 20 and 120 are generally made of a transparent, rigid medical plastic such as polycarbonate, and cannula 28 is typically made of stainless steel.

Accordingly, it can now be understood that there is provided by this invention a cannula and hub assembly which assists in the insertion of cannulas through tough or fibrous tissues within the body of a patient that is easy to use, simple to manufacture and inexpensive.

While the present invention has been disclosed in connection with certain embodiments thereof, it should be understood that there may be other embodiments which fall within the spirit and scope of the invention as defined by the following claims.

CLAIMS:

1. A device for assisting in the positioning of a pointed cannula comprising:

a hub member connected to said cannula at the end opposite to its pointed end;

a removable placement member having first and second projecting segments and a connecting section extending between said first and second projecting segments;

said first and second projecting segments having substantially planar surfaces positioned in a substantially transverse manner with respect to said connecting section; and

a recess positioned in said hub member in a manner substantially transverse to the cannula, said removable placement member constructed and arranged for frictional fitment within said recess;

whereby said removable placement member may be placed within said recess so that said projecting segments are substantially transverse to the cannula.

2. The device as defined in Claim 1 wherein said first and second projecting segments are wing-like members.

3. The device as defined in Claim 1 wherein said removable placement member and the recess within the hub of the cannula and hub assembly include compatible latch means of the frictional fitment type.

4. An injection device comprising:

a cannula having a pointed end;

a hub member connected to said cannula opposite said pointed end;

a removable placement member having first and second projecting segments and a connecting section extending between said first and second projecting segments;

said first and second projecting segments having substantially planar surfaces positioned in a substantially transverse manner with respect to said connecting section; and

a recess positioned in said hub member in a manner substantially transverse to said cannula, said removable placement member constructed and arranged for frictional fitment within said recess;

whereby said removable placement member may be placed within said recess so that said projecting segments are substantially transverse to said cannula.

5. The injection device as defined in Claim 4 wherein said cannula is adapted for use in administering fluids to the epidural space.

6. The injection device as defined in Claim 4 wherein said removable placement member and said hub member include compatible latch means of the frictional fitment type.

7. The injection device as defined in Claim 4 wherein said substantially planar surfaces include depressions.

8. The injection device as defined in Claim 4 wherein the removable placement member is reversible with respect to the hub member.

FIG. 1

FIG. 2

2/2

FIG. 3